# EUROPEAN PATENT APPLICATION

(11) **EP 3 106 183 A1**
(43) Date of publication of application: **21.12.2016**
(21) Application number: 16001271.2
(22) Date of filing: 06.06.2016
(51) Int. Cl.: A61L 27/18, A61L 27/34, A61L 27/56, A61L 27/58

(54) **SCAFFOLD FOR TISSUE ENGINEERING AND METHOD OF FABRICATING THE SAME**

(30) Priority: 18.06.2015 JP 2015123276
(71) Applicant: GC Corporation, Bunkyo-ku Tokyo 113-0033 (JP)
(72) Inventor: Yamanaka, Katsuyuki, Tokyo, 174-8585 (JP); Sakai, Yuuhiro, Tokyo, 174-8585 (JP); Shigemitsu, Yusuke, Tokyo, 174-8585 (JP)
(74) Representative: Müller-Boré & Partner Patentanwälte PartG mbB

(57) **Abstract**

A scaffold for tissue engineering includes a frame structure including one of poly-D-lactic acid and poly-L-lactic acid, and a coating layer formed on a surface of the frame structure and including a lactic acid-glycolic acid copolymer.

## Description

### BACKGROUND OF THE INVENTION

### 1. Field of the Invention

An aspect of this disclosure relates to a scaffold for tissue engineering and a method of fabricating the scaffold.

### 2. Description of the Related Art

There exists a treatment where body tissue lost due to, for example, surgery or an injury is regenerated using somatic cells or stem cells, and the body tissue is transplanted into a patient to recover the lost body tissue. To regenerate body tissue in this treatment, a scaffold (matrix) is necessary to support inoculated cells until the body tissue is regenerated by the cells.

For example, Japanese Laid-Open Patent Publication No. 2006-136673 discloses a block-shaped scaffold for tissue engineering made of a bioabsorbable polymer. The disclosed block-shaped scaffold for tissue engineering has a three-dimensional porous open-pore structure with a pore diameter of 5 to 50 µm and irregular interconnected pores that occupy 20 to 80% of the cross-sectional area of the block-shaped scaffold for tissue engineering. Also, the block-shaped scaffold for tissue engineering has an elastic modulus of 0.1 to 2.5 MPa, and a modulus of volume change of 95 to 105% when immersed in water for 24 hours. Japanese Laid-Open Patent Publication No. 2006-136673 also discloses polyglycolic acid and polylactic acid as examples of bioabsorbable polymers.

Compared with a sponge-like scaffold for tissue engineering, the disclosed block-shaped scaffold for tissue engineering has a higher elastic modulus that provides excellent shape stability, and does not greatly change in shape even when it absorbs water.

Here, there is a demand for a scaffold for tissue engineering that can maintain its structure even when used to culture and differentiate cells over a long time, and has an excellent cell differentiation potency.

### SUMMARY OF THE INVENTION

In an aspect of this disclosure, there is provided a scaffold for tissue engineering that includes a frame structure including one of poly-D-lactic acid and poly-L-lactic acid, and a coating layer formed on a surface of the frame structure and including a lactic acid-glycolic acid copolymer.

### BRIEF DESCRIPTION OF THE DRAWINGS

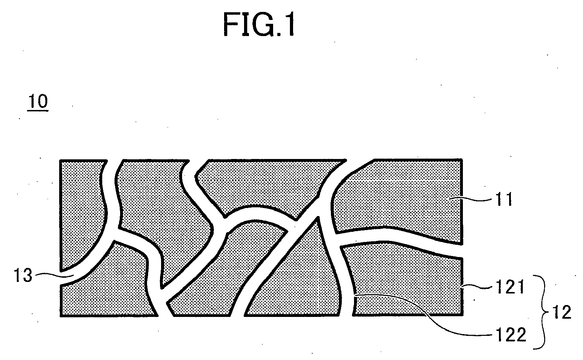

FIG. 1 is a schematic cross-sectional view of a scaffold for tissue engineering according to an embodiment.

### DESCRIPTION OF THE PREFERRED EMBODIMENTS

According to research conducted by the inventors of the present invention, a block-shaped scaffold for tissue engineering fabricated according to Japanese Laid-Open Patent Publication No. 2006-136673 by using polylactic acid as a bioabsorbable polymer can maintain its structure even when used to culture cells over a long time.

However, according to the research, a block-shaped scaffold for tissue engineering fabricated by using polylactic acid as a bioabsorbable polymer does not always have a sufficient cell differentiation potency.

Embodiments of the present invention are described below. However, the present invention is not limited to those embodiments, and variations and modifications may be made without departing from the scope of the present invention.

### «SCAFFOLD FOR TISSUE ENGINEERING»

An exemplary configuration of a scaffold for tissue engineering according to an embodiment is described below.

The scaffold for tissue engineering of the present embodiment may include a frame structure including poly-D-lactic acid or poly-L-lactic acid, and a coating layer formed on a surface of the frame structure and including lactic acid-glycolic acid copolymer.

Components of the scaffold for tissue engineering of the present embodiment are described below.

As described above, the scaffold for tissue engineering of the present embodiment may include a frame structure including poly-D-lactic acid or poly-L-lactic acid.

The frame structure has a three-dimensional porous open-pore structure including irregular interconnected pores. According to research conducted by the inventors of the present invention, including poly-D-lactic acid or poly-L-lactic acid in a frame structure makes it possible to improve the strength of the frame structure and to achieve long-term structural stability of the frame structure during cell culture.

As a type of polylactic acid, poly-DL-lactic acid, which includes both D-lactic acid and L-lactic acid at the same time in the molecular chain, is also known. However, compared with a frame structure including poly-D-lactic acid or poly-L-lactic acid, a frame structure including poly-DL-lactic acid does not always have sufficient long-term structural stability during cell culture. For this reason, the frame structure of the present embodiment preferably includes poly-D-lactic acid or poly-L-lactic acid. More preferably, the frame structure of the present embodiment is made of poly-D-lactic acid or poly-L-lactic acid.

Next, the coating layer is described. The coating layer may include lactic acid-glycolic acid copolymer. According to research conducted by the inventors of the present invention, when used for the frame structure, lactic acid-glycolic acid copolymer provides less long-term structural stability during cell culture compared with poly-D-lactic acid and poly-L-lactic acid. However, because lactic acid-glycolic acid copolymer has excellent capability in holding cells to be cultured, including lactic acid-glycolic acid copolymer in the coating layer coating the surface of the frame structure makes it possible to improve the differentiation potency of the scaffold for tissue engineering.

More preferably, the coating layer is made of lactic acid-glycolic acid copolymer. However, because the coating layer can be formed by immersing the frame structure in a solution containing lactic acid-glycolic acid copolymer and freeze-drying the frame structure, the coating layer may include an inevitable component such as a solvent included in the solution.

Next, an exemplary structure of the scaffold for tissue engineering of the present embodiment is described. FIG. 1 is a schematic cross-sectional view of the scaffold for tissue engineering of the present embodiment.

As described above, the scaffold for tissue engineering has a three-dimensional porous open-pore structure including irregular interconnected pores. In FIG. 1, for illustration purposes, the size of the irregular interconnected pores with respect to the size of the cross section is larger than their actual size. The actual size of the interconnected pores is very small.

As illustrated by FIG. 1, a scaffold for tissue engineering 10 of the present embodiment includes a frame structure 11 and a coating layer 12 formed on a surface (s) of the frame structure 11. The frame structure 11 includes irregular interconnected pores 13.

As illustrated by FIG. 1, the coating layer 12 may include an outer coating layer 121 formed on an outer surface(s) of the frame structure 11, as well as an inner coating layer 122 formed on an inner surface(s) of the interconnected pores 13 in the frame structure 11.

Preferably, the coating layer 12 includes the outer coating layer 121 formed on the entire outer surface of the frame structure 11, and the inner coating layer 122 formed on the entire inner surface of the interconnected pores 13 in the frame structure 11.

The coating layer 12 is preferably, but is not limited to, a porous layer or a sponge-like layer that can effectively hold cells to be cultured.

The proportion of the coating layer 12 to the scaffold for tissue engineering 10 is not limited to any specific value, and can be determined based on, for example, the type or purpose of cells to be cultured. However, to improve the differentiation potency during cell culture, the amount of the coating layer 12 in the scaffold for tissue engineering 10 is preferably greater than or equal to 0.01 mass%, and more preferably greater than or equal to 0.1 mass%.

The maximum amount of the coating layer 12 in the scaffold for tissue engineering 10 is not limited to any specific value. However, the amount of the coating layer 12 in the scaffold for tissue engineering 10 is preferably less than or equal to 2 mass%, and more preferably less than or equal to 1 mass%.

As described above, the scaffold for tissue engineering 10 of the present embodiment includes the frame structure 11 having long-term structural stability during cell culture, and the coating layer 12 that is formed on a surface of the frame structure 11 and can facilitate the differentiation of cells. Thus, the scaffold for tissue engineering 10 of the present embodiment has both long-term structural stability during cell culture and excellent cell differentiation potency.

### «METHOD OF FABRICATING SCAFFOLD FOR TISSUE ENGINEERING»

An exemplary method of fabricating the scaffold for tissue engineering of the present embodiment is described below.

The method of fabricating the scaffold for tissue engineering of the present embodiment may include the following processes:
- a frame structure preparation process of preparing a frame structure including poly-D-lactic acid or poly-L-lactic acid;
- an immersion process of immersing the frame structure in a solution containing lactic acid-glycolic acid copolymer; and
- a freeze-drying process of taking out the frame structure from the solution containing lactic acid-glycolic acid copolymer, and freeze-drying the frame structure.

Each process is described in detail below.

### <FRAME STRUCTURE PREPARATION PROCESS>

First, the frame structure preparation process is described. In the frame structure preparation process, a frame structure including poly-D-lactic acid or poly-L-lactic acid is prepared.

The frame structure preferably has a three-dimensional porous open-pore structure including irregular interconnected pores.

The frame structure may be prepared as described below.

First, poly-D-lactic acid or poly-L-lactic acid is dissolved in an organic solvent (dissolution step). Any organic solvent that can dissolve poly-D-lactic acid or poly-L-lactic acid may be used. For example, the organic solvent preferably includes at least one of chloroform, dichloromethane, carbon tetrachloride, acetone, dioxane, and tetrahydrofuran.

When dissolving poly-D-lactic acid or poly-L-lactic acid in the organic solvent, heat treatment or ultrasonic treatment may also be performed. The concentration of poly-D-lactic acid or poly-L-lactic acid in a solution obtained by dissolving poly-D-lactic acid or poly-L-lactic acid is not limited to any specific value, and may be determined so that poly-D-lactic acid or poly-L-lactic acid can be uniformly dispersed in the organic solvent. Preferably, the amount of poly-D-lactic acid or poly-L-lactic acid in the organic solvent is greater than or equal to 1 mass% and less than or equal to 20 mass%.

Next, particulates are added to and mixed with the solution containing poly-D-lactic acid or poly-L-lactic acid (particulate addition step). The particulates do not dissolve in the organic solvent in the solution, but dissolve in a liquid that does not dissolve poly-D-lactic acid or poly-L-lactic acid.

The particulates are preferably, but are not limited to, a water-soluble organic/inorganic salt such as sodium chloride, potassium chloride, calcium chloride, ammonium chloride, or trisodium citrate.

The diameter of the particulates is preferably greater than or equal to 100 µm and less than or equal to 2000 µm, and more preferably greater than or equal to 200 µm and less than or equal to 1000 µm.

The amount of added particulates with respect to the solution containing poly-D-lactic acid or poly-L-lactic acid is not limited to any specific value, and may be determined according to the required density of a scaffold for tissue engineering to be fabricated. The concentration of the particulates in the solution containing poly-D-lactic acid or poly-L-lactic acid is preferably greater than or equal to 1.0 g/cm³ and less than or equal to 1.5 g/cm³, and more preferably greater than or equal to 1.0 g/cm³ and less than or equal to 1.25 g/cm³.

With the concentration of the particulates set at a value greater than or equal to 1.0 g/cm³, a poly-D-lactic acid/poly-L-lactic acid structure including the particulates becomes very hard, and this in turn makes it easier to grind the poly-D-lactic acid/poly-L-lactic acid structure in a grinding step described later. With the concentration of the particulates set at a value less than or equal to 1.5 g/cm³, the proportion of poly-D-lactic acid or poly-L-lactic acid in a poly-D-lactic acid/poly-L-lactic acid structure including the particulates obtained after a freeze-drying step (described later) becomes sufficiently high, and this in turn makes it possible to increase the yield.

For example, the particulates may be substantially uniformly mixed with the solution containing poly-D-lactic acid or poly-L-lactic acid by adding the particulates to the solution, agitating the solution as necessary, and then pouring the solution into a mold. Also, the particulates may be mixed with the solution containing poly-D-lactic acid or poly-L-lactic acid by pouring the solution into a mold containing the particulates. Further, the particulates may be mixed with the solution containing poly-D-lactic acid or poly-L-lactic acid by putting the particulates into a mold containing the solution.

Next, the solution containing the particulates and poly-D-lactic acid or poly-L-lactic acid is frozen and then dried to remove the organic solvent (freeze-drying step).

As a result of the freeze-drying step, a porous poly-D-lactic acid/poly-L-lactic acid structure including the particulates is obtained. At this stage, the particulates are included in the poly-D-lactic acid/poly-L-lactic acid structure in the form of solid.

Next, the poly-D-lactic acid/poly-L-lactic acid structure including the particulates is ground into granules (grinding step). Because the particulates exist in the poly-D-lactic acid/poly-L-lactic acid structure in the form of solid (particles), the poly-D-lactic acid/poly-L-lactic acid structure becomes hard and can be easily ground into granules with a desired diameter.

Next, from the granules obtained by grinding the poly-D-lactic acid/poly-L-lactic acid structure including the particulates, the particulates are removed by using a solution that does not dissolve poly-D-lactic acid or poly-L-lactic acid (particulate removing step).

The method of removing the particulates may vary depending on the substance forming the particulates. However, when the particulates are formed of a water-soluble organic/inorganic salt such as sodium chloride, potassium chloride, calcium chloride, ammonium chloride, or trisodium citrate, the particulates can be dissolved and removed with water.

After removing the particulates, the granules of poly-D-lactic acid/poly-L-lactic acid (hereafter "poly-D-lactic acid/poly-L-lactic acid granules") may be screened (or sieved) to obtain poly-D-lactic acid/poly-L-lactic acid granules with a desired diameter.

The diameter of the poly-D-lactic acid/poly-L-lactic acid granules supplied to a pressurizing-heating step described later is preferably greater than or equal to 100 µm and less than or equal to 3000 µm.

The scaffold for tissue engineering of the present embodiment includes interconnected pores so that cells can be held in the interconnected pores. This configuration makes it possible to improve the efficiency of cell culture. According to research conducted by the inventors of the present invention, a number of interconnected pores sufficient to improve the efficiency of cell culture can be formed in the scaffold for tissue engineering by making the diameter of the poly-D-lactic acid/poly-L-lactic acid granules supplied to the pressurizing-heating step greater than or equal to 100 µm and less than or equal to 3000 µm.

The porous poly-D-lactic acid/poly-L-lactic acid granules obtained through the above steps are placed in a desired mold, and pressurized and heated to produce a frame structure (pressurizing-heating step).

Although the pressurization condition in this step may vary depending on the shape and size of the frame structure to be produced, the pressurization condition is preferably greater than or equal to 500 g/cm² and less than or equal to 3000 g/cm², and more preferably greater than or equal to 1000 g/cm² and less than or equal to 2000 g/cm².

Setting the pressurization condition at a value greater than or equal to 500 g/cm² makes it possible to improve the shape stability of the frame structure, and setting the pressurization condition at a value less than or equal to 3000 g/cm² makes it possible to form a sufficient number of interconnected pores for growing cells.

The heating condition in this step may also vary depending on the shape and size of the frame structure. When the poly-D-lactic acid/poly-L-lactic acid granules are to be heated under the above pressurization condition while maintaining their volume, the heating condition is preferably greater than or equal to 60 °C and less than or equal to 200 °C. Heating the poly-D-lactic acid/poly-L-lactic acid granules at a temperature greater than or equal to 60 °C makes it possible to increase the bond between the poly-D-lactic acid/poly-L-lactic acid granules, and thereby makes it possible to improve the shape stability of the frame structure. Also, heating the poly-D-lactic acid/poly-L-lactic acid granules at a temperature less than or equal to 200 °C makes it possible to prevent denaturation of poly-D-lactic acid or poly-L-lactic acid.

### <IMMERSION PROCESS>

In the immersion process, the frame structure prepared in the frame structure preparation process is immersed in a solution containing lactic acid-glycolic acid copolymer.

By immersing the frame structure in the solution containing lactic acid-glycolic acid copolymer, surfaces of the frame structure can be substantially uniformly covered with the solution containing lactic acid-glycolic acid copolymer.

As described above, the frame structure has a three-dimensional porous open-pore structure including irregular interconnected pores. Therefore, by immersing the frame structure in the solution containing lactic acid-glycolic acid copolymer, not only the outer surface of the frame structure but also the inner surface of the interconnected pores formed in the frame structure can be covered with the solution containing lactic acid-glycolic acid copolymer.

Any solvent that can dissolve lactic acid-glycolic acid copolymer may be used to prepare the solution containing lactic acid-glycolic acid copolymer. However, a solvent that hardly dissolve poly-D-lactic acid or poly-L-lactic acid included in the frame structure is preferable.

That is, a solvent that can dissolve lactic acid-glycolic acid copolymer but can hardly dissolve poly-D-lactic acid or poly-L-lactic acid is preferably used to prepare the solution containing lactic acid-glycolic acid copolymer. For example, the solution containing lactic acid-glycolic acid copolymer preferably includes a solvent including one or more of acetone, dioxane, and tetrahydrofuran. The solvent of the solution containing lactic acid-glycolic acid copolymer particularly preferably includes 1,4-dioxane.

The concentration of lactic acid-glycolic acid copolymer in the solution is not limited to any specific value, and may be determined taking into account, for example, the thickness of the coating layer to be formed and operability. The concentration of lactic acid-glycolic acid copolymer in the solution is preferably greater than or equal to 0.01 mass% and less than or equal to 10 mass%, and more preferably greater than or equal to 0.01 mass% and less than or equal to 7 mass%. Setting the concentration of lactic acid-glycolic acid copolymer at a value greater than or equal to 0.01 mass% makes it possible to reliably form the coating layer on the entire surface of the frame structure. Setting the concentration of lactic acid-glycolic acid copolymer at a value less than or equal to 10 mass% makes it possible to keep the viscosity of the solution at a low level, and to improve the operability of the solution in the immersion process.

The time period for which the frame structure is immersed in the solution containing lactic acid-glycolic acid copolymer in the immersion process is not limited to any specific value, and may be determined depending on, for example, the size of the frame structure. However, the time period is preferably determined such that the surface of the frame structure is not excessively dissolved by the solution containing lactic acid-glycolic acid copolymer, and still the surface of the frame structure can be sufficiently brought into contact with the solution containing lactic acid-glycolic acid copolymer. The temperature of the solution containing lactic acid-glycolic acid copolymer may also be determined at any value depending on the type of the solvent. For example, when the solvent is 1,4-dioxane, the temperature of the solution is preferably between 15 and 20 °C.

### <FREEZE-DRYING PROCESS>

In the freeze-drying process performed after the immersion process, the frame structure is taken out of the solution containing lactic acid-glycolic acid copolymer, and the frame structure is freeze-dried.

In the freeze-drying process, the frame structure immersed in the solution containing lactic acid-glycolic acid copolymer is frozen to prevent the frame structure from being dissolved by the solvent in the solution and to remove the solvent by drying. As a result, only the solvent is removed from the solution containing lactic acid-glycolic acid copolymer applied to the surface of the frame structure in the immersion process, and lactic acid-glycolic acid copolymer remains on the surface as a coating layer. Accordingly, the coating layer becomes a porous layer. Forming a porous coating layer on the frame structure makes it possible to improve the capability of the scaffold for tissue engineering to hold cells to be cultured.

The freezing condition in the freeze-drying process is not limited to any specific condition. However, the freeze-drying process is preferably performed at a temperature less than or equal to -20 °C, and more preferably less than or equal to -30 °C In view of productivity, the temperature for freezing is preferably greater than or equal to -100 °C.

A scaffold for tissue engineering fabricated by the method of the present embodiment described above includes a frame structure having long-term structural stability during cell culture, and a coating layer that is formed on a surface of the frame structure and can facilitate the differentiation of cells. Thus, the method of the present embodiment can fabricate a scaffold for tissue engineering that has both long-term structural stability during cell culture and excellent cell differentiation potency.

### «EXAMPLES»

Examples below are provided to facilitate understanding of the present invention. However, the present invention is not limited to those examples.

### <EXAMPLE 1>

In Example 1, a scaffold for tissue engineering was fabricated as described below.

### (FRAME STRUCTURE PREPARATION PROCESS)

A frame structure preparation process was performed as described below to prepare a frame structure including poly-L-lactic acid.

First, poly-L-lactic acid (about 250,000 weight-average molecular weight) was added to 1,4-dioxane such that the concentration of poly-L-lactic acid would become 6 mass%, and the resulting mixture was agitated with an agitator at 70 °C to dissolve poly-L-lactic acid and obtain a 1,4-dioxane solution containing poly-L-lactic acid (dissolution step).

Next, trisodium citrate powder (with a particle diameter between 200 µm to 500 µm) was substantially uniformly mixed with the 1,4-dioxane solution containing poly-L-lactic acid such that the concentration of trisodium citrate powder would become about 1.02 g/cm³ (particulate addition step), and the resulting solution was poured into a mold.

The solution was frozen at -30 °C using a freezer (SANYO Electric Co., Ltd., product name: MDF-0281AT). Then, the frozen solution was dried for 48 hours under reduced pressure using a vacuum dryer (Yamato Scientific Co., Ltd., product name: DP43) to remove 1,4-dioxane and obtain a polymer of poly-L-lactic acid substantially uniformly containing trisodium citrate powder (freeze-drying step).

The polymer was cut into pieces, and the pieces were ground for 20 minutes using a planetary pot mill (grinding step).

The ground polymer material was put into a flask, distilled water was added to the flask, and the resulting mixture was agitated to remove trisodium citrate powder (particulate removing step). After removing trisodium citrate powder, the ground polymer material was placed in a petri dish and was dried for 48 hours using a vacuum drier. Then, the ground and dried polymer material was screened to obtain porous poly-L-lactic acid granules with a diameter between 700 µm and 1400 µm and an average pore diameter of about 5 µm.

The obtained porous poly-L-lactic acid granules were placed in a glass container with an inside diameter of 9 mm and a height of 10 mm up to a height of about 7 mm. The porous poly-L-lactic acid granules were heated at 180 °C for 30 minutes, while maintaining the volume of the porous poly-L-lactic acid granules being pressurized at 1500 g/cm² by a titanium rod having a diameter of 9 mm (pressurizing-heating step), to obtain a frame structure.

The obtained frame structure was observed with an electron microscope, and it was confirmed that the frame structure had a three-dimensional porous open-pore structure having pores in partitioning walls and also including irregular interconnected pores.

### (IMMERSION PROCESS)

Next, an immersion process was performed to immerse the obtained framework structure in a solution containing lactic acid-glycolic acid copolymer.

Using 1,4-dioxane as a solvent, a solution containing 0.2 mass% of lactic acid-glycolic acid copolymer was prepared.

The entire frame structure was immersed in the prepared solution containing lactic acid-glycolic acid copolymer for 20 seconds at 18 °C.

### (FREEZE-DRYING PROCESS)

Next, a freeze-drying process was performed by taking out the frame structure from the solution containing lactic acid-glycolic acid copolymer, and freeze-drying the frame structure.

In the freeze-drying process, the frame structure was frozen at -30 °C using a freezer (SANYO Electric Co., Ltd., product name: MDF-0281AT). Then, the frozen frame structure was dried for 48 hours under reduced pressure using a vacuum dryer (Yamato Scientific Co., Ltd., product name: DP43) to remove 1,4-dioxane and obtain a scaffold for tissue engineering.

The amount of a coating layer including lactic acid-glycolic acid copolymer in the scaffold for tissue engineering was obtained based on the weight of the frame structure before the immersion process and the weight of the scaffold for tissue engineering after the freeze-drying process. The amount of the coating layer was 0.75 mass%.

### <EXAMPLE 2>

In Example 2, a scaffold for tissue engineering was fabricated in substantially the same manner as in Example 1 except that a solution containing 0.07 mass% of lactic acid-glycolic acid copolymer was prepared using 1,4-dioxane as a solvent in the immersion process.

The amount of the coating layer including lactic acid-glycolic acid copolymer in the fabricated scaffold for tissue engineering was obtained in a manner similar to Example 1. The amount of the coating layer was 0.29 mass%.

### <EXAMPLE 3>

In Example 3, a scaffold for tissue engineering was fabricated in substantially the same manner as in Example'1 except that a frame structure including poly-D-lactic acid was used.

The frame structure including poly-D-lactic acid was prepared in substantially the same manner as in Example 1 except that in the dissolution step of the frame structure preparation process, poly-D-lactic acid (about 250,000 weight-average molecular weight) was added to 1,4-dioxane such that the concentration of poly-D-lactic acid would become 10 mass% and the resulting mixture was agitated with an agitator.

The amount of the coating layer including lactic acid-glycolic acid copolymer in the fabricated scaffold for tissue engineering was obtained in a manner similar to Example 1. The amount of the coating layer was 0.63 mass%.

An aspect of this disclosure makes it possible to provide a scaffold for tissue engineering that has both long-term structural stability during cell culture and excellent cell differentiation potency, and a method of fabricating such a scaffold for tissue engineering.

A scaffold for tissue engineering and a method of fabricating the scaffold for tissue engineering according to embodiments are described above. However, the present invention is not limited to the specifically disclosed embodiments, and variations and modifications may be made without departing from the scope of the present invention.

## Claims

1. A scaffold for tissue engineering, comprising:
a frame structure including one of poly-D-lactic acid and poly-L-lactic acid; and
a coating layer formed on a surface of the frame structure and including a lactic acid-glycolic acid copolymer.

2. The scaffold for tissue engineering as claimed in claim 1, wherein an amount of the coating layer in the scaffold for tissue engineering is greater than or equal to 0.01 mass% and less than or equal to 2 mass%.

3. A method of fabricating a scaffold for tissue engineering, the method comprising:
preparing a frame structure including one of poly-D-lactic acid and poly-L-lactic acid;
immersing the frame structure in a solution containing a lactic acid-glycolic acid copolymer; and
taking out the frame structure from the solution containing the lactic acid-glycolic acid copolymer and freeze-drying the frame structure.

4. The method as claimed in claim 3, wherein the solution containing the lactic acid-glycolic acid copolymer includes a solvent including one or more of acetone, dioxane, and tetrahydrofuran.
